# EUROPEAN PATENT APPLICATION

(11) **EP 4 382 032 A1**
(43) Date of publication of application: **12.06.2024**
(21) Application number: 22842466.9
(22) Date of filing: 13.07.2022
(51) Int. Cl.: A61B 3/10, A61B 3/00, A61B 5/00

(54) **CASE-TYPE PORTABLE OTC DEVICE WITH DISPLAY HAVING DOOR STRUCTURE**

(30) Priority: 13.07.2021 KR 20210091361
(71) Applicant: Philophos, Inc., Daejeon 34137 (KR)
(72) Inventor: CHUNG, Jung Ho, Yongin-si Gyeonggi-do 16805 (KR)
(74) Representative: Manitz Finsterwald Patent- und Rechtsanwaltspartnerschaft mbB
(86) International application number: PCT/KR2022/010230
(87) International publication number: WO 2023/287204

(57) **Abstract**

A portable optical coherence tomography (OCT) system according to one embodiment of the present invention comprises: a main body in which an optical part and a control part of an OCT device are mounted, and which includes a stepped part having a protruding lower front side so that an examinee can load, through the stepped part, an area to be examined, and allows a light source of the optical part to be incident on the area to be examined of the examinee through a hole formed in the front upper side at which the stepped part is disposed; a display part disposed such that a screen for displaying an image captured by the optical part of the OCT device faces a side surface part of the main body; and at least one display movement part connected to one edge of the main body and one end of the display part so that position of the display part moves, wherein the display part rotates with respect to the display movement part so as to display an image of the examinee according to the exposure of the screen to an examiner, and re-rotates by means of the display movement part in order to store same, so as to be rearranged such that the screen faces the side surface part of the main body.

## Description

### FIELD OF THE TECHNOLOGY DISCLOSED

The present disclosure relates to a case-type portable Optical Coherence Tomography (OCT) device with a display having a door structure.

### BACKGROUND

OCT is an advanced medical diagnostic technology that enables the inside of a biological tissue to be observed by using light and has been used in the fields of ophthalmology, cardiovascular medicine, and so on. The OCT can be implemented by such configurations as time domain, spectral domain, and swept source. Particularly, the spectral domain OCT system ensures a certain level of performance and requires less manufacturing cost and thus has received a lot of attention.

In a conventional OCT system, there was a problem that each device constituting the system was large in size, and research to miniaturize each device has been ongoing.

However, in an OCT device developed through such efforts, there is a problem that the OCT device has a structure in which a display device provided independently from a separate Personal Computer (PC) is connected to the separate PC, and each of the display device and the PC are disposed on a desk and are used. Therefore, the OCT device is very inconvenient to be carried, and is mounted such that a rear surface or a side surface of the OCT device is exposed, so that there is a problem that the OCT device is exposed to an external shock when the external shock occurs. In addition, in a conventional OCT, there is a problem that an examiner is required to directly adjust an adjustment standard with a joystick, and there is an inconvenience of carrying, moving, and storing a face mount for mounting an examinee's face and a probe part of an OCT device since the face mount and the probe part are mechanically separated.

### SUMMARY

the present disclosure has been made keeping in mind the above problems occurring in the related art, and an objective of the present disclosure is to provide an OCT device in which a main body and a display device of an OCT system are provided as an integrated type and which is capable of being easily carried.

In order to achieve the above objectives, according to an embodiment of the present disclosure, there is provided a portable Optical Coherence Tomography (OCT) system including: a main body in which an optical part and a control part of an OCT device are mounted, the main body including a stepped part having a protruding lower front side so that an examinee is capable of loading, through the stepped part, an area to be examined, and the main body allowing a light source of the optical part to be incident on a sample of the examinee through a hole formed in a front upper side at which the stepped part is disposed; a display part disposed such that a screen for displaying an image captured by the optical part of the OCT device faces a side surface part of the main body; and at least one display movement part connected to one edge of the main body and one end of the display part so that a position of the display part is capable of being moved, wherein the display part is configured to be rotated with respect to the display movement part so as to display an image of the examinee according to an exposure of the screen to an examiner, and is configured to be re-rotated by the display movement part in order to store the display part, so as to be rearranged such that the screen faces the side surface part of the main body.

In addition, the display movement part may be formed as a hinge, the display part may be configured to be moved to a rear surface part of the main body by the hinge, and a center of the display part may be in close contact with the rear surface part of the main body.

In addition, the main body may further include: a slide panel formed such that the slide panel is disposed in close contact with the rear surface part of the main body, the slide panel being mounted such that the slide panel is capable of being optionally slid in a direction parallel to a plane of the rear surface part of the main body; and a rail part mounted on the rear surface part of the main body and configured to slide the slide panel. Furthermore, the display movement part may be mounted on an edge of the slide panel, the display part may be configured to be slid in a direction parallel to the plane of the rear surface part of the main body by the rail part and the slide panel after the display part is rotated by the display movement part.

In addition, the portable OCT system may further include a clasp structure and a protrusion part that are formed between the rear surface part of the main body and the slide panel. When the display part is disposed in close contact with the side surface part of the main body, the clasp structure and the protrusion part may be coupled to each other, thereby preventing the slide panel from being moved. When the display part is rotated by the display movement part and is disposed such that the display part faces the rear surface part of the main body, coupling between the clasp structure and the protrusion part may be released, thereby allowing the slide panel to be moved.

In addition, the display part may be disposed such that the display part is positioned on a center of the rear surface part of the main body by being slid by the slide panel after the display part is folded at least 90 degrees by the hinge.

In addition, the portable OCT system may further include a display fixing part configured to fix the display part to a rear surface part of the main body after the display part is rotated toward the rear surface part of the main body.

In addition, the display fixing part may include: a door catch having a clasp structure formed on the display part and a protrusion part corresponding to the clasp structure, the protrusion part being mounted on the rear surface part of the main body; or a magnetic structure mounted on each of the display part and the rear surface of the main body.

In addition, the portable OCT system may further include a cover part formed in a structure capable of covering a structure for loading the examinee's area to be examined.

In addition, the portable OCT system may further include a second rail part formed such that the second rail part extends to one region of an upper surface of the main body from one region of an edge where a front surface part and the upper surface part of the main body 100 meet, wherein a first side region of the cover part and the second rail part may be coupled to each other, and the cover part may be moved to the upper surface part of the main body by being slid on the front surface part of the main body.

In addition, a chin rest part and a forehead fixing part that are configured to fix the examinee's face may be further provided on the front surface part of the main body, and a louver fixing part having a structure in which the chin rest part and the forehead fixing part are capable of being detached from and attached to the structure may be further provided on one region of the cover part.

In addition, the chin rest part may include: a chin rest supporting part formed vertically at a predetermined length on an upper surface of the stepped part; and a rest part horizontally fixed to a first side of the chin rest supporting part, the rest part having a pair of plate structures that has a predetermined area. Furthermore, each plate of the rest part may have a predetermined curvature and may be configured in a shape symmetrical to each other, and a center of the rest part and the chin rest supporting part may be coupled to each other.

In addition, the chin rest part may have a structure capable of being detached from and attached to the main body.

In addition, the rest part may have a structure in which two sides of the pair of plate structures having short lengths are capable of being folded with respect to the center of the rest part such that the two sides are in contact with each other, and may have a structure in which any one of the plate structures is capable of being pulled in a vertical direction after the plate structure is pulled in a horizontal direction so that a shaft supporting the rest part is capable of being moved.

In addition, the portable OCT system may further include a forehead fixing part having a plate structure that protrudes from a front surface part of the main body on one region of the front surface part of the main body, the forehead fixing part having a structure capable of being detached from and attached to the main body.

In addition, the forehead fixing part may have a rectangular structure, may have a predetermined one region of a side having a short length coupled to the main body, and may be configured to be rotated around a part coupled to the main body.

In addition, a main body supporting part having a plate structure formed at a predetermined length and a predetermined width may be further provided on a lower surface of the main body, and one region of the main body supporting part and one region of a lower surface part of the main body may be coupled to each other.

In addition, the main body supporting part may be configured such that the one region of the main body supporting part coupled to the main body is rotated so that the main body supporting part protrudes to an outside of the main body.

In addition, there is provided a method for providing a portable Optical Coherence Tomography (OCT) device, the portable OCT device including: a main body in which an optical part and a control part of the OCT device are mounted, the main body being configured such that a light source of the optical part is incident to a sample of an examinee through a hole formed in one region of the main body; a display part disposed such that a screen of the display part faces a side surface part of the main body, the display part being configured to display an image captured by the optical part of the OCT device; and at least one display movement part connected to one edge of the main body and one end of the display part so that a position of the display part is capable of being moved, the method including: (a) a process in which the portable OCT device is prepared and a main body supporting part is unfolded; (b) a process in which the screen of the display part is exposed by rotating the display part by using the display movement part; (c) a process in which a cover coupled one region of the main body is released such that a stepped part of the main body configured to load an examined area of the examinee thereon and the hole connected to the optical part are exposed; (d) a process in which the optical part and the control part in the main body are operated by pushing a power button of the main body, thereby displaying the examinee's image on the display part; and (e) a process of changing a state of the portable OCT device into a state in which the portable OCT device is capable of being easily carried by reversely performing the (a) process to the (d) process after an examination is performed.

### BRIEF DESCRIPTION OF THE DRAWINGS

FIG. 1A is a first view illustrating a configuration of a portable Optical Coherence Tomography (OCT) system according to an embodiment of the present disclosure.
FIG. 1B is a second view illustrating the configuration of the portable OCT system according to an embodiment of the present disclosure.
FIG. 2A is a view illustrating a first embodiment of a method in which a display part is folded toward a rear surface according to an embodiment of the present disclosure.
FIG. 2B is a view illustrating the first embodiment of the method in which the display part is folded toward the rear surface according to an embodiment of the present disclosure.
FIG. 2C is a view illustrating the first embodiment of the method in which the display part is folded toward the rear surface according to an embodiment of the present disclosure.
FIG. 3A is a view illustrating a second embodiment of a method in which the display part is folded toward the rear surface according to an embodiment of the present disclosure.
FIG. 3B is a view illustrating the second embodiment of the method in which the display part is folded toward the rear surface according to an embodiment of the present disclosure.
FIG. 4A is a view illustrating an example of a structure of a cover part of the OCT system according to an embodiment of the present disclosure.
FIG. 4B is a view illustrating the example of the structure of the cover part of the OCT system according to an embodiment of the present disclosure.
FIG. 4C is a view illustrating the example of the structure of the cover part of the OCT system according to an embodiment of the present disclosure.
FIG. 5A is an example view illustrating a first embodiment of storing a chin rest part and a forehead fixing part according to an embodiment of the present disclosure.
FIG. 5B is an example view illustrating the first embodiment of storing the chin rest part and the forehead fixing part according to an embodiment of the present disclosure.
FIG. 6A is an example view illustrating a second embodiment of storing the chin rest part and the forehead fixing part according to an embodiment of the present disclosure.
FIG. 6B is an example view illustrating the second embodiment of storing the chin rest part and the forehead fixing part according to an embodiment of the present disclosure.
FIG. 7A is a view illustrating a first embodiment of a position adjustment function of the chin rest part according to an embodiment of the present disclosure.
FIG. 7B is a view illustrating the first embodiment of the position adjustment function of the chin rest part according to an embodiment of the present disclosure.
FIG. 8A is a view illustrating a second embodiment of the position adjustment function of the chin rest part according to an embodiment of the present disclosure.
FIG. 8B is a view illustrating the second embodiment of the position adjustment function of the chin rest part according to an embodiment of the present disclosure.
FIG. 9A is a view illustrating a position adjustment function of the forehead fixing part according to an embodiment of the present disclosure.
FIG. 9B is a view illustrating the position adjustment function of the forehead fixing part according to an embodiment of the present disclosure.
FIG. 10A is a view illustrating a structure of a main body supporting part according to an embodiment of the present disclosure.
FIG. 10B is a view illustrating the structure of the main body supporting part according to an embodiment of the present disclosure.

### DETAILED DESCRIPTION

Hereinafter, an embodiment of the present disclosure will be described in detail with reference to the accompanying drawings such that a person having ordinary knowledge in the technical field to which the present disclosure belongs may easily implement the embodiment of the present disclosure. However, it is understood that the present disclosure is able to be implemented in various different forms and is not limited to the embodiment described herein. In addition, parts in the drawings unrelated to the detailed description are omitted to ensure clarity of the present disclosure, and like reference numerals in the drawings denote like elements throughout.

Throughout the specification, it will be understood that when an element is referred to as being "connected" to another element, it can be directly connected to the other element or it can be electrically connected with the other element and intervening elements may be present therebetween. In addition, it will be further understood that when a part "comprises", "includes", or "has" an element, this means that other elements are not excluded but may be further included, unless otherwise stated. It will be further understood that the terms "comprises", "includes", or "has" used in this specification, specify the presence of stated features, steps, operations, components, parts, or a combination thereof, but do not preclude the presence or addition of one or more other features, numerals, steps, operations, components, parts, or a combination thereof.

Following embodiments described hereinafter are detailed descriptions of the present disclosure to facilitate understanding of the present disclosure and are not intended to limit the scope of the present disclosure. Thus, a subject matter having the same scope and performing the same function as those of the present disclosure also fall within the protection scope of the present disclosure.

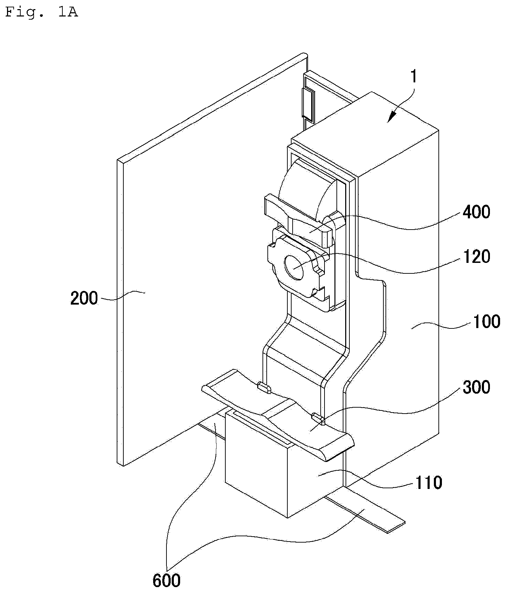

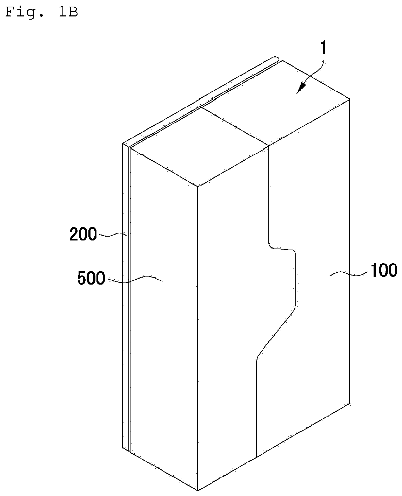

FIG. 1A and FIG. 1B are views illustrating a configuration of a portable Optical Coherence Tomography (OCT) system 1 according to an embodiment of the present disclosure.

Referring to FIG. 1A, the portable OCT system 1 includes a main body 100 in which an OCT device is mounted, a display part 200, a chin rest part 300, a forehead fixing part 400, and a main body supporting part 600.

In the specification of the present disclosure, the OCT device is implemented as a miniaturized module and is placed inside the main body 100. Furthermore, the OCT device includes an optical part, a circuit part, a power source part, and so on that are constituting the OCT device, and is configured to independently capture and process optical coherence tomography.

According to an embodiment of the present disclosure, the portable OCT system 1 is implemented as an OCT device placed in the main body 100 having a polyhedral shape. At this time, the display part 200 is provided on a side surface of the main body 100, and an image captured through the OCT device is displayed on the display part 200.

In addition, the display part 200 may be provided such that a screen displaying an image captured by the optical part of the OCT device faces a side surface part of the main body 100. At this time, although not illustrated in FIG. 1A, at least one display movement part connected to one edge of the main body 100 and one end of the display part 200 such that a position of the display part 200 is capable of being moved may be provided.

Accordingly, the display part 200 is configured to be rotated with respect to the display movement part so as to display an image of an examinee according to the exposure of the screen to an examiner, and is configured to be re-rotated by means of the display movement part in order to store the same, so as to be rearranged such that the screen faces the side surface part of the main body 100.

Specifically, each structure of the OCT system 1 is described as follows.

According to an embodiment of the present disclosure, the main body 100 has a hexahedral structure in which the OCT device is mounted, and one region of the hexahedral structure is recessed. The chin rest part 300 and the forehead fixing part 400, which will be described later, are disposed on the recessed region, and an examination or a measurement of the examinee is performed on the recessed region.

That is, the optical part and a control part of the OCT device is mounted inside the main body 100, and the main body 100 includes a stepped part 110 having a protruding lower front side as illustrated in FIG. 1A. In addition, the main body 100 has a structure in which the examinee is capable of loading, through the stepped part 110, an area to be examined, and allows a light source of the optical part to be incident on a sample of the examinee through a hole formed in an upper front side at which the stepped part 110 is disposed.

At this time, the hole is formed in a first side region of a front surface part of the main body 100, and a lens part 120 of the OCT device is formed in the corresponding hole so that the lens part 120 is exposed to the outside. In addition, as described above, the stepped part 110 having a predetermined size is formed on a second side region of the front surface part of the main body 100, and the stepped part 110 is configured to mount the chin rest part 300.

According to an embodiment of the present disclosure, the display part 200 is disposed in close contact with the side surface part of the main body 100, and serves to provide a tomographic image captured by the optical part of the OCT device to the examiner.

In addition, as an additional embodiment, the display part 200 may be provided with a touch screen having an input and output function, so that the display part 200 may serve as an operation part that allows a user to operate the OCT device.

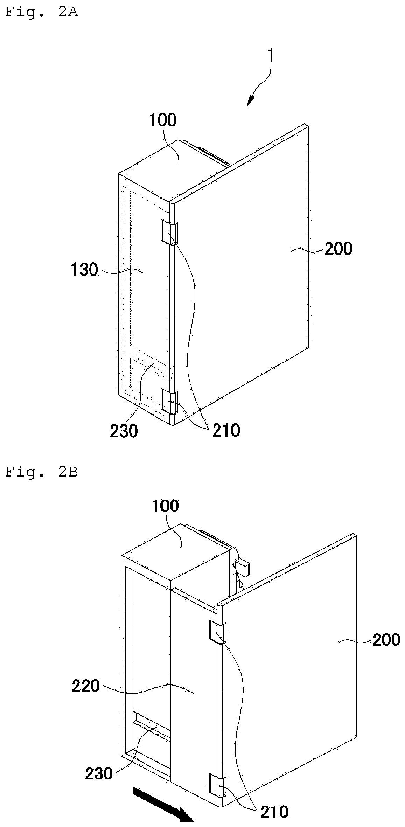

Meanwhile, at least one display movement part 210 (for example, a hinge (specifically illustrated in FIG. 2A and FIG. 2B)) connected to one edge of the main body 100 and one end of the display part 200 such that a position of the display part 200 is capable of being moved is included.

Therefore, the display part 200 may be folded at least 90 degrees with respect to an edge between the side surface part of the main body 100 and a rear surface part 130 of the main body 100 so that the display part 200 is positioned such that the display part 200 is in close contact with the rear surface part 130 of the main body 100, and may display an image. A specific situation in which the display part 200 is folded will be described again through two embodiments to be described later.

Therefore, the user may position the display part 200 on a side surface of the OCT system 1 or on a rear surface of the OCT system 1 for convenience of use, so that the examiner may view the screen together with a patient or may view the screen alone as required. At this time, the display part 200 is moved to the rear surface part 130 of the main body 100, and a center of the display part 200 is positioned such that the center of the display part 200 is fixed to a center of the rear surface part 130 of the main body 100.

According to an embodiment of the present disclosure, the chin rest part 300 may be formed on an upper surface of the stepped part 110 that is formed on the main body 100. The chin rest part 300 is implemented for the purpose of supporting the chin of the examinee, and may be formed in various shapes. Among the various shapes of the chin rest part 300, the chin rest part 300 may be formed in a T-shape structure. Therefore, the patient may support the patient's chin on the chin support part 300 and may align a position of the patient's eye to the lens part 120, and then an OCT image may be captured.

According to an embodiment of the present disclosure, similar to the chin rest part 300, the forehead fixing part 400 is intended to be used to assist in capturing by fixing the patient's forehead.

Therefore, the forehead fixing part 400 may be formed on one region of the front surface part of the main body 100 facing the stepped part 110 of the main body 100, and may be formed in a plate structure having a surface parallel to the main body 100.

Next, referring to FIG. 1B, a hexahedral structure in which a cover part 500 covers the front surface part of the main body 100 is illustrated.

According to an embodiment of the present disclosure, when the cover part 500 is coupled to the main body 100, the stepped part 110, the lens part 120, the chin rest part 300, and the forehead fixing part 400 located at the front surface part of the main body 100 are protected.

At this time, in FIG. 1B, it is illustrated that the cover part 500 covers the front surface of the main body 100. However, as an optional embodiment, the stepped part 110 may be exposed so that only the lens part 120, the chin rest part 300, and the forehead fixing part 400 may only be protected.

Meanwhile, the OCT device may be operated by performing following five processes.

As a first process, the main body supporting part 600 may be unfolded after the portable OCT device is prepared.

As a second process, by using the display movement part 210, the display part 200 may be rotated such that the screen of the display part 200 is exposed.

In a third process, a cover coupled to one region of the main body 100 may be released, and the stepped part 110 of the main body 100 configured such that the examinee is capable of loading an area to be examined and the hole connected to the optical part may be exposed.

In a fourth process, as a power button of the main body 100 is pushed, the optical part and the control part in the main body 100 are operated, so that an image of the examinee may be displayed on the display part 200.

As a final process, after an examination process is performed, the display part 200 may be re-rotated by the display movement part 210 so as to be stored, and the screen may be rearranged such that the screen faces the side surface part of the main body 100. That is, by reversing the first to fourth processes, the portable OCT device may be transformed into a state in which the portable OCT device is capable of being easily moved (stored).

FIGS. 2A to 2C are views illustrating a first embodiment of a method in which the display part 200 is folded toward a rear surface according to an embodiment of the present disclosure.

In the first embodiment, a process in which the display part 200 is moved to the rear surface part 130 of the main body 100 is described in the order of FIGS. 2A to 2C.

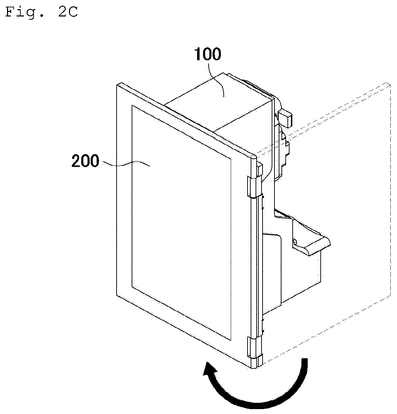

At this time, referring to FIG. 2B and FIG. 2C, in the OCT system 1, the display movement part 210 may be provided between the rear surface part 130 and the side surface part of the main body 100. At this time, the display movement part 210 may be formed as a hinge, the display part 200 may be moved to the rear surface part 130 of the main body 100 by the hinge, and the center of the display part 200 may be in close contact with the rear surface part 130 of the main body 100.

In addition, a slide panel 220 may be mounted on the main body 100, the slide panel 220 being formed such that the slide panel 220 is in close contact with the rear surface part 130 of the main body 100, and the slide panel being mounted such that the slide panel 220 is capable of being optionally slid in a direction parallel to a plane of the rear surface part 130 of the main body 100. In addition, a rail part 230 is further provided, the rail part 230 being mounted on the rear surface part 130 of the main body 100 and being configured such that the slide panel 220 is capable of being slid.

At this time, in the embodiment illustrated in FIG. 2A, the hinge corresponding to the display movement part 210 may be mounted such that an edge of the slide panel 220 and an edge of the display part 200 are connected to each other.

Therefore, after the display part 200 is rotated by the display movement part 210, the display part 200 is moved in the direction parallel to the plane of the rear surface part 130 of the main body 100 by the rail part 230 and the slide panel 220, and the display part 200 is disposed such that the center of the display part 200 is in contact with the center of the rear surface part 130 of the main body 100.

At this time, as an optional additional embodiment, the rail part 230 may be provided in the form of a groove formed from the side surface part of the main body 100 toward the inside of the main body 100. In this case, the slide panel 220 may have a structure inserted into and fixed to the inside of the main body 100.

Through this, in a normal situation, the display part 200 is disposed on the side surface of the main body 100 as illustrated in FIG. 2A, so that the OCT system 1 has a rectangular shape such that the volume of the OCT system 1 is minimized. At this time, there is an advantage that the examiner may conveniently carry and transport the OCT system 1. However, when the examiner intends to perform the examination on the examinee, the examinee is located in the direction of the stepped part 110. When the display part 200 is positioned on the side surface of the main body 100, it is difficult for the examiner to look at the display part 200 and the examinee at the same time. Therefore, the display part 200 is capable of being moved to the rear surface part 130 of the main body 100 by sliding the display part 200 as illustrated in FIG. 2C, so that the examiner may conveniently diagnose and measure the examinee.

Although not illustrated in the drawings, a clasp structure and a protrusion part that are formed between the rear surface part 130 and the slide panel 220 of the main body 100 may be further included. At this time, when the display part 200 is disposed such that the display part 200 is in close contact with the side surface part of the main body 100, the clasp structure and the protrusion part are coupled to each other, so that a movement of the slide panel 220 may be prevented. In addition, when the display part 200 is rotated by the display movement part 210 and is disposed such that the display part 200 faces the rear surface part 130 of the main body 100, the coupling between the clasp structure and the protrusion part are released, so that the slide panel 220 is capable of being moved. At this time, as mentioned above, after the display part 200 is folded at least 90 degrees by the hinge, the display part 200 may be positioned at the rear surface part 130 of the main body 100 by being slid by the slide panel 220.

Meanwhile, as another embodiment, a display fixing part may be further included, the display fixing part being configured to fix the display part 200 to the rear surface part 130 of the main body 100 after the display part 200 is rotated toward the rear surface part 130 of the main body 100. At this time, the display fixing part may correspond to a door catch having a clasp structure formed on the display part 200 and may correspond to a protrusion part which is mounted on the rear surface part 130 of the main body 100 and which corresponds to the clasp structure, or the display fixing part may correspond to magnetic structures respectively formed on the display part 200 and the rear surface part 130 of the main body 100.

In addition, various embodiments of fixing the rear surface part 130 of the main body 100 and the display part 200 to each other, which may be easily derived by those skilled in the art, may be included in an embodiment of the present disclosure.

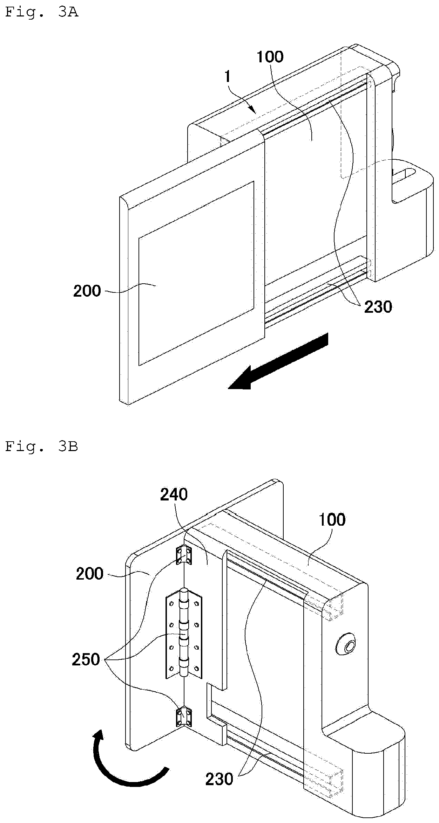

FIG. 3A and FIG. 3B are views illustrating a second embodiment of a method in which the display part is folded toward the rear surface according to an embodiment of the present disclosure.

Referring to FIG. 3A, the rail part 230 may be formed such that the rail part 230 horizontally extends on upper and lower ends of the side surface part of the main body 100. In this case, a second slide panel 240 is coupled to the rail part 230, and is coupled to the rear surface part of the display part 200 by a second hinge 250.

Accordingly, as illustrated in FIG. 3A, the display part 200 may be implemented such that the display part 200 is pulled backward parallel to the side surface of the main body 100 and then the display part 200 is folded backward and fixed as illustrated in FIG. 3B.

At this time, as the display part 200 completely overlaps the side surface part of the main body 100, the display part 200 is prevented from protruding. Therefore, as an optional embodiment, the size of the display part 200 may be smaller than the size of the side surface part of the main body 100. However, the display part 200 does not necessarily correspond to a shape of the side surface part of the main body 100. In addition, in the display part 200, a region where an image is displayed faces an outside surface of the display part 200.

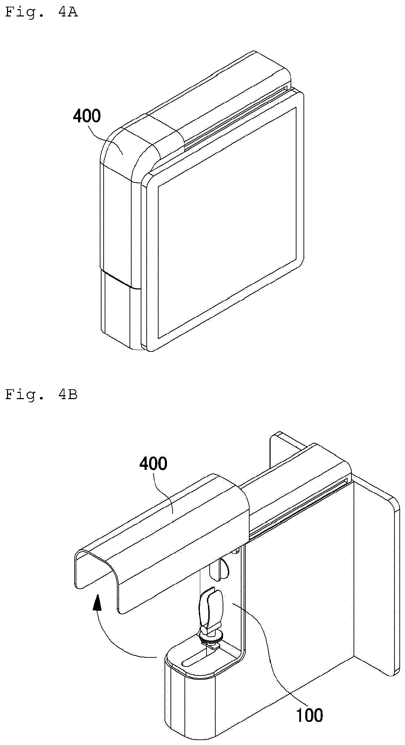

FIG. 4A and FIG. 4B are views illustrating an example of a structure of the cover part 500 of the OCT system 1 according to an embodiment of the present disclosure.

Prior to the description, the cover part 500 may be formed in a structure capable of covering a structure for loading the examined area of the examinee. For example, the cover part 500 may be formed in a structure capable of covering the lens part 120 where a light source is emitted, the chin rest part 300, the forehead fixing part 400, and the stepped part 110.

At this time, as a first embodiment, the cover part 500 has a structure capable of being detached from and attached to the front surface part of the main body 100. Accordingly, the cover part 500 has a plate structure having a U-shaped predetermined curved surface that can cover the lens part 120, the forehead fixing part 400, and the chin rest part 300. That is, when the cover part 500 is coupled to the main body 100, the cover part 500 completely covers the chin rest part 300 and the forehead fixing part 400 as illustrated in FIG. 4A.

Next, as a second embodiment, the cover part 500 may be implemented in a non-detachable form. Specifically, a second rail part may further be formed, the second rail part being formed such that the second rail part extends to one region of an upper surface of the main body 100 from one region of an edge where the front surface part and the upper surface part of the main body 100 meet.

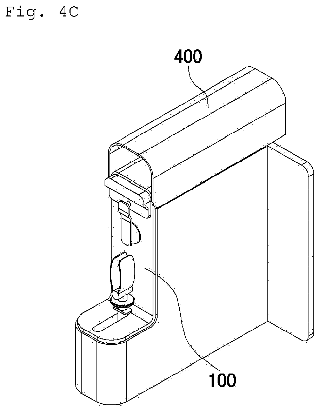

At this time, a first side region of the cover part 500 and the second rail part are coupled to each other, the cover part 500 is slid from the front surface part of the main body 100, and the cover part 500 is moved to the upper surface part of the main body 100. For example, as illustrated in FIG. 4A, the cover part 500 coupled to the front surface part of the main body 100 is coupled such that the first side region of the cover part 500 is coupled to the second rail part as illustrated in FIG. 4B, and the cover part 500 is moved to the upper surface part of the main body 100 by being slid on the front surface part of the main body 100 as illustrated in FIG. 4C.

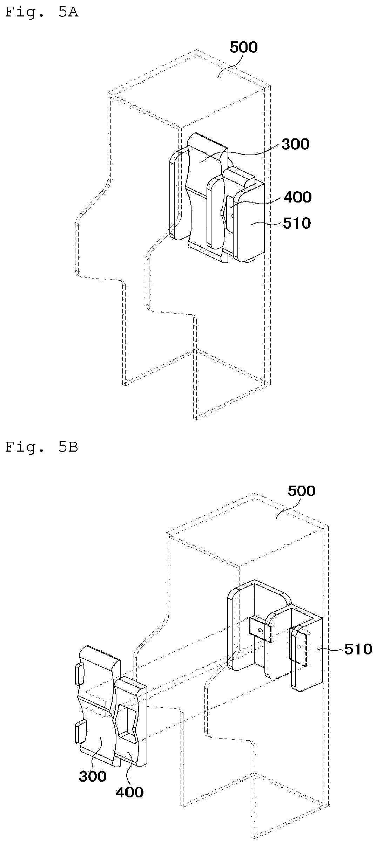

FIG. 5A and FIG. 5B are example views illustrating a first embodiment of storing the chin rest part 300 and the forehead fixing part 400 according to an embodiment of the present disclosure.

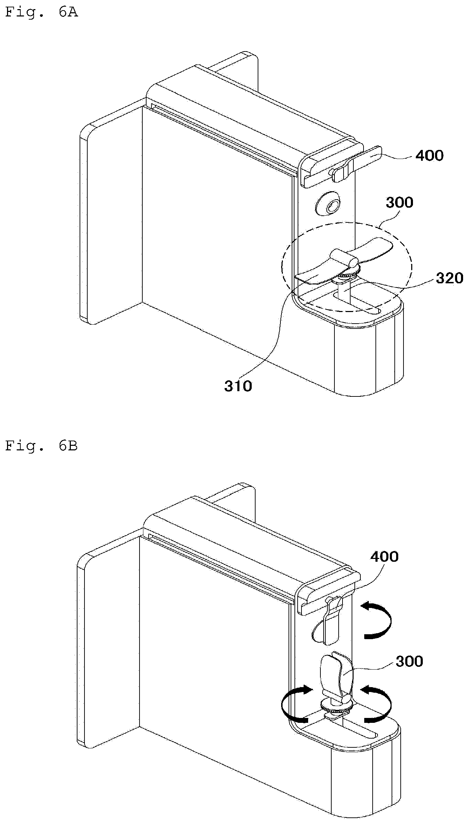

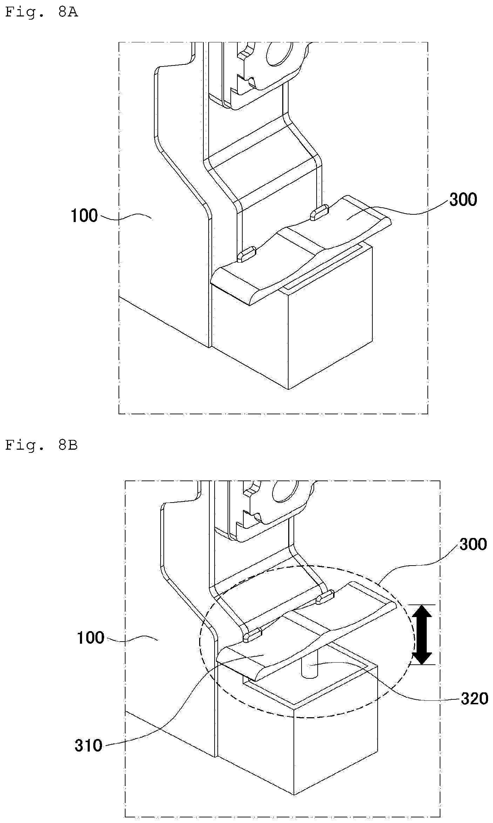

Prior to the description, referring to FIG. 6A and FIG. 8B, the chin rest part 300 includes a chin rest supporting part 320 that is vertically formed at a predetermined length on an upper surface of the stepped part 110, and includes a rest part 310 which is horizontally fixed to a first side of the chin rest supporting part 320 and which has a pair of plate structures having a predetermined area.

At this time, each plate of the rest part 310 has a predetermined curvature and is configured to be symmetrical with each other, and a center of the rest part 310 is coupled to the chin rest supporting part 320.

As an optional embodiment, the rest part 310 has a W-shaped shape having a predetermined curvature and having a symmetrical shape, and the chin rest supporting part 320 is coupled to the center of the rest part 310 so that a 'T' shape is maintained. Therefore, according to a direction of the eye being measured, the patient may support the chin on any one region of the rest part 310 in which the curvature of the 'W' shape is formed.

For example, in a situation in which the left eye is measured, the chin and the forehead are fixed by placing the chin and the forehead on a right side of the chin rest part 300 and the forehead fixing part 400, thereby being capable of measuring the left eye. In a situation in which the right eye is measured, the chin and the forehead are fixed by placing the chin and the forehead on a left side of the chin rest part 300 and the forehead fixing part 400, thereby being capable of measuring the right eye.

In addition, the forehead fixing part 400 has a plate structure that protrudes from the front surface part of the main body 100 on one region of the front surface part of the main body 100, and is implemented such that the forehead fixing part 400 has a structure that is capable of being detached from and attached to the main body 100.

For example, the forehead fixing part 400 has a rectangular plate structure, and a predetermined curvature is formed such that short sides of the forehead fixing part 400 are facing each other, so that the forehead fixing part 400 may stably support the forehead.

Again, in describing the first embodiment again, it is assumed that the chin rest part 300 and the forehead fixing part 400 have structures capable of being detached from and attached to the main body 100.

At this time, referring to FIG. 5A and FIG. 5B, a louver fixing part 510 may be further provided inside the cover part 500, the louver fixing part 510 corresponding to each of the chin rest part 300 and the forehead fixing part 400 that are detached, and the louver fixing part 510 being capable of being coupled to each of the chin rest part 300 and the forehead fixing part 400.

Therefore, before the cover part 500 of the OCT system 1 that completed the examination is coupled to the main body 100, the chin rest part 300 and the forehead fixing part 400 that are separated from the main body 100 are coupled to the louver coupling part 510 that is formed inside the cover part 500.

FIG. 6A and FIG. 6B are example views illustrating a second embodiment of storing the chin rest part 300 and the forehead fixing part 400 according to an embodiment of the present disclosure.

In describing the second embodiment with reference to FIG. 6A, the rest part 310 of the chin rest part 300 may have a structure in which the two sides having short lengths are capable of being folded such that the two sides are in contact with each other with respect to the center of the pair of plate structures. At this time, in order to remove discontinuity of a surface in contact with the chin, a structure in which any one of the plate structure is capable of being pulled in a vertical direction after the plate structure is pulled in a horizontal direction may be formed, so that a shaft supporting the rest part 310 is capable of being moved and then folded.

In addition, the forehead fixing part 400 has a rectangular structure, and a predetermined one region of any one side having a short length of the forehead fixing part 400 is coupled to the main body 100. At this time, the forehead fixing part 400 may be provided such that the forehead fixing part 400 is capable of being rotated around a part coupled to the main body 100. Alternatively, as a bow tie, the center of the forehead fixing part 400 may be coupled to the main body 100, so that the forehead fixing part 400 may have a structure in which the forehead fixing part 400 is capable of being rotated 90 degrees.

That is, in the OCT system 1 that has been used, as illustrated in FIG. 6B, the rest part 310 of the chin rest part 300 is folded to the center and the forehead fixing part 400 is vertically rotated, so that a state in which the cover part 500 is capable of covering the chin rest part 300 and the forehead fixing part 400 is provided.

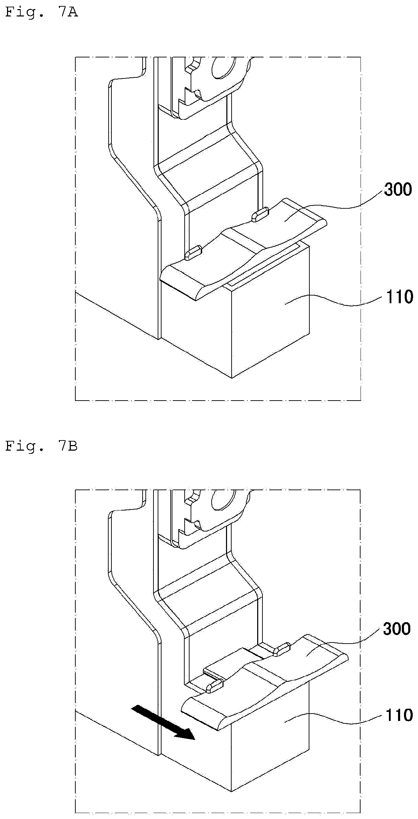

FIG. 7A and FIG. 7B are views illustrating a first embodiment of a position adjustment function of the chin rest part 300 according to an embodiment of the present disclosure.

Referring to FIG. 7A, a 1-shaped groove is formed in the upper surface of the stepped part 110 toward a front side of the device, and the chin rest part 300 is coupled to the groove.

Therefore, as illustrated in FIG. 7B, the chin rest part 300 may be moved forward and backward along the groove, and a depth may be adjusted according to the patient's face.

FIG. 8A and FIG. 8B are views illustrating a second embodiment of the position adjustment function of the chin rest part 300 according to an embodiment of the present disclosure.

In describing the second embodiment with reference to FIG. 8A, a level of the chin rest part 300 may be adjusted as illustrated in FIG. 8B by adjusting a level of the chin rest supporting part 320 that constitutes the chin rest part 300. Through this, an eye level of the examinee is capable of being adjusted to a level of the lens part 120.

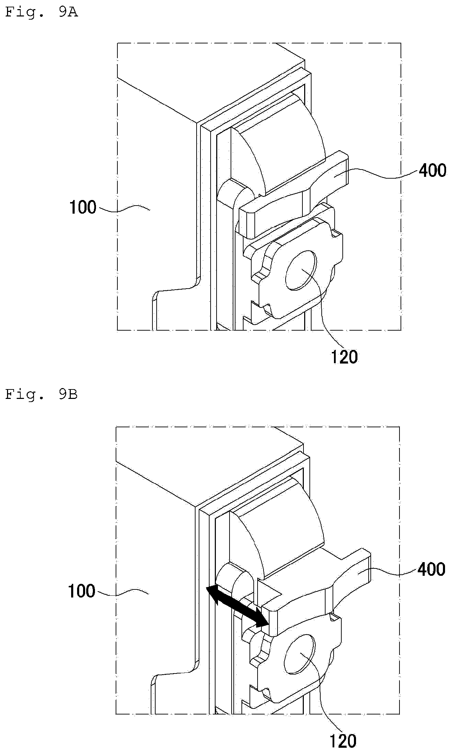

FIG. 9A and FIG. 9B are views illustrating a position adjustment function of the forehead fixing part 400 according to an embodiment of the present disclosure.

Referring to FIG. 9A, the forehead fixing part 400 may be provided such that the forehead fixing part 400 is in close contact with the main body 100.

Furthermore, referring to FIG. 9B, the forehead fixing part 400 may protrude in a direction facing the front surface part of the main body 100 so as to fit the forehead of the examinee.

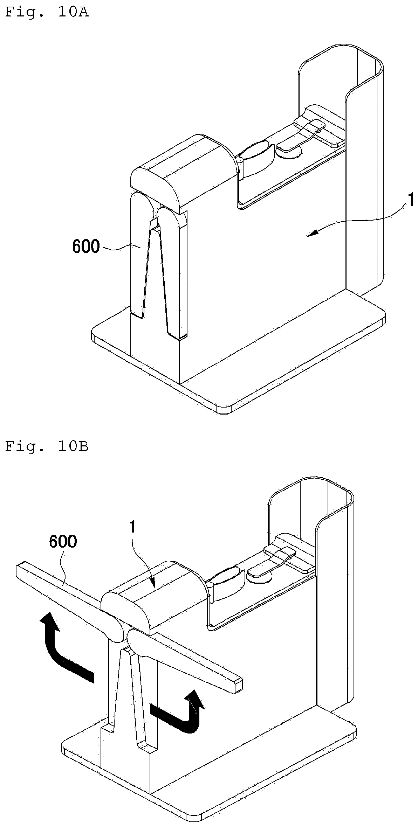

FIG. 10A and FIG. 10B are views illustrating a structure of the main body supporting part 600 according to an embodiment of the present disclosure.

Referring to FIG. 10A, the main body supporting part 600 having a plate structure formed at a predetermined length and a predetermined width is further provided on a lower surface of the main body 100. At this time, one region of the main body supporting part 600 and one region of the lower surface of the main body 100 are coupled to each other.

In addition, one region of the main body supporting part 600 coupled to the main body 100 is rotated, so that the main body supporting part 600 protrudes outside the main body 100.

That is, the main body supporting part 600 may be hidden on the lower surface of the main body 100 as illustrated in FIG. 10A, or the main body supporting part 600 may spread like a wing as illustrated in FIG. 10B.

Through this, when the OCT system 1 is placed upright, the main body supporting part 600 supports the OCT system 1 so that the OCT system 1 does not fall.

At this time, as an additional embodiment, a material having an uneven shape or having a high frictional force may be provided in one region of the main body supporting part 600 that is in contact with the ground. Through this, the OCT system 1 is prevented from slipping.

Meanwhile, as an additional embodiment, a predetermined hole may be provided in one region of the upper surface part of the main body 100, and a handle part for the user to lift the OCT system 1 may be provided in the hole. At this time, the handle part may be provided as a detachable type handle part, or may be provided in a form in which the handle part is pushed into or pulled out from the hole.

In the OCT device according to an embodiment of the present disclosure, a display device and the main body of the OCT system are provided as an integrated type, and the display device is provided such that the display device is capable of being folded into the main body, so that a volume of the OCT device is minimized and the OCT device is very convenient to be carried and transported.

In addition, a foldable structure may be applied to a required part such as the display device or a face mount, so that the volume of the OCT device may be minimized, thereby allowing the OCT device to be transported. In addition, since a leg part supporting the main body is optionally provided and a monitor is capable of being moved, the main body is properly supported during an actual measurement, so that a structure in which an examinee and an examiner are capable of conveniently performing an examination may be provided.

Although embodiments of the present disclosure have been described for illustrative purposes, those skilled in the art will appreciate that various modifications, additions and substitutions are possible, without changing the spirit or essential features of the present disclosure. Therefore, it should be understood that, in every aspect, the embodiments described above are examples and are not restrictive. For example, any component described as having an integrated form may be implemented in a distributed form, and any component described as having a distributed form may also be implemented in an integrated form.

The scope of the present disclosure is represented by the following claims, rather than the detailed description, and it is to be construed that the meaning and scope of the claims and all variations or modified forms derived from the equivalent concept thereof are encompassed within the scope of the present disclosure.

## Claims

1. A portable Optical Coherence Tomography (OCT) system comprising:
a main body in which an optical part and a control part of an OCT device are mounted, the main body comprising a stepped part having a protruding lower front side so that an examinee is capable of loading, through the stepped part, an area to be examined, and the main body allowing a light source of the optical part to be incident on a sample of the examinee through a hole formed in a front upper side at which the stepped part is disposed;
a display part disposed such that a screen for displaying an image captured by the optical part of the OCT device faces a side surface part of the main body; and
at least one display movement part connected to one edge of the main body and one end of the display part so that a position of the display part is capable of being moved,
wherein the display part is configured to be rotated with respect to the display movement part so as to display an image of the examinee according to an exposure of the screen to an examiner, and is configured to be re-rotated by the display movement part in order to store the display part, so as to be rearranged such that the screen faces the side surface part of the main body.

2. The portable OCT system of claim 1, wherein the display movement part is formed as a hinge, the display part is configured to be moved to a rear surface part of the main body by the hinge, and a center of the display part is in close contact with the rear surface part of the main body.

3. The portable OCT system of claim 2, wherein the main body further comprises:
a slide panel formed such that the slide panel is disposed in close contact with the rear surface part of the main body, the slide panel being mounted such that the slide panel is capable of being optionally slid in a direction parallel to a plane of the rear surface part of the main body; and
a rail part mounted on the rear surface part of the main body and configured to slide the slide panel, and
the display movement part is mounted on an edge of the slide panel, the display part is configured to be slid in a direction parallel to the plane of the rear surface part of the main body by the rail part and the slide panel after the display part is rotated by the display movement part.

4. The portable OCT system of claim 3, further comprising a clasp structure and a protrusion part that are formed between the rear surface part of the main body and the slide panel,
wherein, when the display part is disposed in close contact with the side surface part of the main body, the clasp structure and the protrusion part are coupled to each other, thereby preventing the slide panel from being moved, and
when the display part is rotated by the display movement part and is disposed such that the display part faces the rear surface part of the main body, coupling between the clasp structure and the protrusion part are released, thereby allowing the slide panel to be moved.

5. The portable OCT system of claim 4, wherein the display part is disposed such that the display part is positioned on a center of the rear surface part of the main body by being slid by the slide panel after the display part is folded at least 90 degrees by the hinge.

6. The portable OCT system of claim 1, further comprising a display fixing part configured to fix the display part to a rear surface part of the main body after the display part is rotated toward the rear surface part of the main body.

7. The portable OCT system of claim 6, wherein the display fixing part comprises:
a door catch having a clasp structure formed on the display part and a protrusion part corresponding to the clasp structure, the protrusion part being mounted on the rear surface part of the main body; or
a magnetic structure mounted on each of the display part and the rear surface of the main body.

8. The portable OCT system of claim 1, further comprising a cover part formed in a structure capable of covering a structure for loading the examinee's area to be examined.

9. The portable OCT system of claim 8, further comprising a second rail part formed such that the second rail part extends to one region of an upper surface of the main body from one region of an edge where a front surface part and the upper surface part of the main body meet,
wherein a first side region of the cover part and the second rail part are coupled to each other, and the cover part is moved to the upper surface part of the main body by being slid on the front surface part of the main body.

10. The portable OCT system of claim 8, wherein a chin rest part and a forehead fixing part that are configured to fix the examinee's face are further provided on the front surface part of the main body, and
a louver fixing part having a structure in which the chin rest part and the forehead fixing part are capable of being detached from and attached to the structure is further provided on one region of the cover part.

11. The portable OCT system of claim 1, wherein the chin rest part comprises:
a chin rest supporting part formed vertically at a predetermined length on an upper surface of the stepped part; and
a rest part horizontally fixed to a first side of the chin rest supporting part, the rest part having a pair of plate structures that has a predetermined area, and
each plate of the rest part has a predetermined curvature and is configured in a shape symmetrical to each other, and a center of the rest part and the chin rest supporting part are coupled to each other.

12. The portable OCT system of claim 11, wherein the chin rest part has a structure capable of being detached from and attached to the main body.

13. The portable OCT system of claim 11, wherein the rest part has a structure in which two sides of the pair of plate structures having short lengths are capable of being folded with respect to the center of the rest part such that the two sides are in contact with each other, and has a structure in which any one of the plate structures is capable of being pulled in a vertical direction after the plate structure is pulled in a horizontal direction so that a shaft supporting the rest part is capable of being moved.

14. The portable OCT system of claim 1, further comprising a forehead fixing part having a plate structure that protrudes from a front surface part of the main body on one region of the front surface part of the main body, the forehead fixing part having a structure capable of being detached from and attached to the main body.

15. The portable OCT system of claim 14, wherein the forehead fixing part has a rectangular structure, has a predetermined one region of a side having a short length coupled to the main body, and is configured to be rotated around a part coupled to the main body.

16. The portable OCT system of claim 1, wherein a main body supporting part having a plate structure formed at a predetermined length and a predetermined width is further provided on a lower surface of the main body, and
one region of the main body supporting part and one region of a lower surface part of the main body are coupled to each other.

17. The portable OCT system of claim 16, wherein the main body supporting part is configured such that the one region of the main body supporting part coupled to the main body is rotated so that the main body supporting part protrudes to an outside of the main body.

18. A method for providing a portable Optical Coherence Tomography (OCT) device, the portable OCT device comprising:
a main body in which an optical part and a control part of the OCT device are mounted, the main body being configured such that a light source of the optical part is incident to a sample of an examinee through a hole formed in one region of the main body;
a display part disposed such that a screen of the display part faces a side surface part of the main body, the display part being configured to display an image captured by the optical part of the OCT device; and
at least one display movement part connected to one edge of the main body and one end of the display part so that a position of the display part is capable of being moved,
the method comprising:
(a) a process in which the portable OCT device is prepared and a main body supporting part is unfolded;
(b) a process in which the screen of the display part is exposed by rotating the display part by using the display movement part;
(c) a process in which a cover coupled one region of the main body is released such that a stepped part of the main body configured to load an examined area of the examinee thereon and the hole connected to the optical part are exposed;
(d) a process in which the optical part and the control part in the main body are operated by pushing a power button of the main body, thereby displaying the examinee's image on the display part; and
(e) a process of changing a state of the portable OCT device into a state in which the portable OCT device is capable of being easily carried by reversely performing the (a) process to the (d) process after an examination is performed.
